# EUROPEAN PATENT SPECIFICATION

(11) **EP 2 377 919 B1**
(45) Date of publication and mention of the grant of the patent: **25.03.2015**
(21) Application number: 10168801.8
(22) Date of filing: 08.07.2010
(51) Int. Cl.: C12Q 1/04, C12N 1/20

(54) **Method for identification of bacteria of the genus Pectinatus and culture medium therefor**
Verfahren und Kulturmedium zur Kultivierung und Identifizierung von Bakterien der Gattung Pectinatus
Procédé et Milieu de culture pour la culture et l'identification de bactéries du genre Pectinatus et procédé d'échantillonnage

(30) Priority: 16.04.2010 CZ 20100297
(43) Date of publication of application: 19.10.2011
(73) Proprietor: Vyzkumny ustav pivovarsky a sladarsky, a.s., 12044 Praha (CZ)
(72) Inventor: Matoulkova, Dagmar, 54101 Trutnov (CZ); Kosar, Karel, 62100 Brno (CZ)
(74) Representative: Musil, Dobroslav

(56) References cited:
- EP-A1- 2 055 766
- US-A- 5 869 642
- US-A1- 2007 254 063
- LEE S Y ET AL: "Selective-differential medium for isolation and differentiation of pectinatus from other brewery microorganisms." APPLIED AND ENVIRONMENTAL MICROBIOLOGY FEB 1981 LNKD- PUBMED:16345712, vol. 41, no. 2, February 1981 (1981-02), pages 386-387, XP002598273 ISSN: 0099-2240
- HAAKENSEN M ET AL: "Broth and agar hop-gradient plates used to evaluate the beer-spoilage potential of Lactobacillus and Pediococcus isolates" INTERNATIONAL JOURNAL OF FOOD MICROBIOLOGY, ELSEVIER SCIENCE PUBLISHERS, AMSTERDAM, NL LNKD- DOI:10.1016/J.IJFOODMICRO.2009.01.001, vol. 130, no. 1, 15 March 2009 (2009-03-15), pages 56-60, XP025946222 ISSN: 0168-1605 [retrieved on 2009-01-09]
- FLAHAUT S ET AL: "Impact of thermal variations on biochemical and physiological traits in Pectinatus sp" INTERNATIONAL JOURNAL OF FOOD MICROBIOLOGY, vol. 55, no. 1-3, 10 April 2000 (2000-04-10), pages 53-61, XP002598274 ISSN: 0168-1605
- SAKAMOTO K ET AL: "Beer spoilage bacteria and hop resistance" INTERNATIONAL JOURNAL OF FOOD MICROBIOLOGY, ELSEVIER SCIENCE PUBLISHERS, AMSTERDAM, NL LNKD- DOI:10.1016/S0168-1605(03)00153-3, vol. 89, no. 2-3, 31 December 2003 (2003-12-31), pages 105-124, XP002538037 ISSN: 0168-1605

## Description

### Technical field

The invention relates to a method for identification of bacteria of the genus *Pectinatus* in a sample of spoilt beer or in a sample taken from a brewery environment and/or from a brewery equipment.

### Background art

Due to modifications in bottling technologies of beer at which the content of oxygen in beer is reduced under the limit of 1 mg/l, increasing production of some sorts of beers, which are, based on their nature predisposed to bacterial contamination, like for example the low-hopped beers, low-alcoholic, non-alcoholic or non-pasteurized beers, and usage of the flow pasteurisation or cold sterilisation of beer, exists at present an increased risk of contamination of beer by anaerobic microorganisms. These microorganisms mostly occur as secondary, i.e. post-pasteurisation contaminants, and they "spoil" the beer, when they produce during their life cycle a number of chemical compounds (propionic acid, acetic acid, hydrogen sulphide, etc.), which negatively influence organoleptic properties of beer, increase its acidity and cause the characteristic massive haze and unpleasant odour. In exceptional cases, due to accumulation of greater quantity of gas produced by these microorganisms, there may even occur explosion of the bottle.

From this point of view, genus *Pectinatus* belongs to the most important anaerobic microorganisms, while the estimates are that at present it causes more than 30% of beer spoilage. These bacteria are tolerant to alcohol up to 4,5% (w/v), to reduced pH up to the value of 3,7 as well as to the hop bitter compounds, and even though that these are strictly anaerobic bacteria, they are able to survive in aerosol for a certain period of time - see e.g. Helander I.M., Haikara A., Sadovskaya I., Vinogradov E., Salkinoja-Salonen M.S. (2004): "Lipopolysaccharides of anaerobic beer spoilage bacteria of the genus Pectinatus - lipopolysaccharides of a gram-positive genus"; FEMS Microbiol. Rev. 28: 543-552.

Presence of bacteria of genus *Pectinatus* in beer and/or in brewery plant or equipment is nevertheless hardly detectable, as due to the specific growing requirements of these bacteria the convention membrane filtration cannot be used for their identification. According to the articles Haikara A. (1984): "Detection of Pectinatus contaminants in beer." J. Am. Soc. Brew. Chem. 43 (1): 43-46, and Haikara A. (1985): "Detection of anaerobic, gram-negative bacteria in beer." Monats. Brauwiss. 6: 239-243 bacteria of genus *Pectinatus* cannot be identified even upon application of membrane filtration, at which the pre-reduced culture medium is used, and which is running in the CO₂ atmosphere.

On the ground of the same reasons it is neither possible to use the cultivation on common culture media on Petri dishes, though a number of national and international institutions recommend this procedure. For example the European Brewing Convention recommends usage of non-selective pre-reduced solidified culture media of the MRS, NBB, PYF, Raka-Ray, SDA and UBA type or the SMMP selective liquid culture medium, see e.g. Analytica Microbiologica - EBC, 2005.

The disadvantage in usage of the non-selective culture media of the above mentioned types is, that this media do not prevent growth of further species of microorganisms being present in a spoilt beer and/or in a brewery environment and/or equipment, while these microorganisms (yeast, coliform bacteria, lactic acid bacteria, etc.) successfully compete with bacteria of genus *Pectinatus* on culture media, suppress their cultivation and possibly inhibit them by products of their metabolism, by change of pH, etc. As a reason of this, the usage of non-selective culture media for cultivation and subsequent identification of bacteria of genus *Pectinatus* is not sufficiently confirmative, hence either suitable for application in common brewing practice.

Application of these culture media is moreover recommended in a solidified pre-reduced form for membrane filtration and cultivation in anaerobic conditions, as a result of which they act reliably only under condition that the given laboratory is equipped with anaerobic chamber with controlled atmosphere of N₂ and H₂, which is nevertheless not common equipment of brewery plants.

Some of these disadvantages are partially remedied by usage of liquid selective culture medium of the SMMP type. Here, thanks to the content of substances reducing the redox potential and antibiotics (e.g. actinon), the growth of yeast is inhibited, nevertheless determining the presence of bacteria of genus *Pectinatus* requires monitoring of the investigated beer, creation of the haze in it and changes in colouring of the culture medium for a period of 14 days. This is period which is totally unsuitable for application in brewery environment. Moreover this culture medium is primary designated only for cultivation and identification of bacteria directly in the spoilt beer and it is not suitable for cultivation of bacteria of genus *Pectinatus* from swab samples taken from brewery equipment and/or in brewery premises.

In the article of Lee S.Y., Moore S.E., Mabee M.S.: "Selective-differential medium for isolation and differentiation of Pectinatus from other brewery microorganisms." Appl. Environ. Microbiol. 2: 386-387, 1981 is for cultivation and subsequent identification of bacteria of genus *Pectinatus* proposed application of so called LL-agar, at which presence of these bacteria is expressed by a black colouring resulting from chemical reaction of hydrogen sulphide produced by bacteria with lead acetate present in the culture medium. The disadvantage of usage of this culture medium is necessity to use so called Lee test-tubes with double wall, which are commonly not available, and related high acquisition costs as well as operational costs. Moreover, this method of cultivation and identification of bacteria of genus *Pectinatus* is relatively time demanding as to preparation (usually 5 to 6 days), and it does not enable detection of these bacteria at their low number in the sample.

To eliminate the above mentioned disadvantages of conventional techniques, in specialised literature and patent documents, e.g. JP 2004121259, JP 2005006556 or US 5869642 were further proposed several methods for identification of bacteria of genus *Pectinatus,* which are based on detection of specific areas of DNA of these bacteria. Nevertheless these methods are demanding both as to time and finances, as for their performance they require not only specialised personnel skilled in complicated molecular techniques, but also a specific laboratory equipment. The identification itself is usually preceded by cultivation and isolation of bacteria, possibly by further steps, as a result of which the whole process may take up to several days. Moreover the outputs of these methods are not clear in most cases, so that there may occur a false positive/negative identification. Due to this even these methods are not suitable for application in common brewery plants.

With respect to the above mentioned disadvantages is for identification of bacteria of genus *Pectinatus* in beer and/or in brewery plants and/or equipment at present used so called "forcing test". Upon this test the sample taken is being inoculated into the beer enriched by substances supporting grow of bacteria of genus *Pectinatus,* and closed bottles are incubated at temperature of 28 to 30 °C. At the same time, within a period of 1 to 6 weeks since inoculation, the formation of haze, which is accompanying effect of presence of the bacteria of genus *Pectinatus,* is being observed. The disadvantages of this procedure are obvious. There belongs especially a long time necessary for cultivation and identification, work difficulty of this procedure, when it is necessary to add the individual enriching components into the beer in a sterile manner, and also increased demands on volume of biological incubators with respect to the size of beer bottles.

The goal of the invention is to propose a method of identification of bacteria of the genus *Pectinatus* in a sample of spoilt beer or in a sample taken from a brewery environment and/or from a brewery equipment, which would be applicable in common brewery plants without necessity to acquire special laboratory equipment, and whose usage would maximally shorten the time necessary for identification of these bacteria. Usage of this method should simultaneously not be limited to identification of bacteria of genus *Pectinatus* in contaminated beer, but it should enable their identification also in the swab samples taken in brewery plants and/or from the brewery equipment.

### Principle of the invention

In its broadest sense, the present invention pertains to subject-matter as defined in the appended claims

The goal of the invention has been achieved by a method of identification of bacteria of the genus *Pectinatus* in a sample of spoilt beer or in a sample taken from a brewery environment and/or from a brewery equipment, whose principle consists in that the sample of spoilt beer or the sample taken from the brewery environment and/or from the brewery equipment is inoculated into a culture medium which comprises at least one source of carbon, at least one source of nitrogen, at least one source of amino-acids, at least one source of vitamins, at least one source of sulphur, at least one source of biogenous metals, at least one substance reducing redox potential, a buffer component and further iso-α-acids and/or their reduced hydrogenated derivatives in concentration of 10 to 80 mg/l. These stimulate by their presence cultivation of bacteria of genus *Pectinatus,* and simultaneously totally or at least partially inhibit cultivation of competing microorganisms. Then, the bacteria of genus *Pectinatus* may be observed and identified as early as after 24 hours after inoculation, this only by means of common microscope.

During experiments, the most suitable concentration of iso-α-acids and/or their reduced hydrogenated derivatives was stated to be 50 mg/l.

In case of need, for example in case of transportation of the samples, the used culture medium further contains 0,05 to 0,3 % by weight of agar, which restricts its oxidation during manipulation and transport.

As reduced hydrogenated derivatives of iso-α-acids the culture medium comprises tetrahydroiso-α-acids and/or dihydroiso-α-acids and/or hexahydroiso-α-acids.

To achieve the best results, during the method according to the invention, it is further advantageous, if in combination with it is simultaneously used a method for taking swab samples by means of swab having sampling head according to the invention. Its principle consists in that, before swab sample taking the sampling head of the swab is dipped into the solution of sterilised destilled water with addition of substance reducing the redox potential in concentration of 0,25 to 3 g/l, and the swab sample taken is positioned into the culture medium which comprises at least one source of carbon, at least one source of nitrogen, at least one source of amino-acids, at least one source of vitamins, at least one source of sulphur, at least one source of biogenous metals, at least one substance reducing redox potential, a buffer component and further iso-α-acids and/or their reduced hydrogenated derivatives in concentration of 10 to 80 mg/l.

As a substance reducing the redox potential there may be used the cysteine hydrochloride, sodium thioglycolate or ascorbic acid in concentration of 0,25 to 1 g/l, possibly mixture of at least two of these substances, while at the same time concentration of either does not exceed the value of 1 g/l.

### Description of the drawing

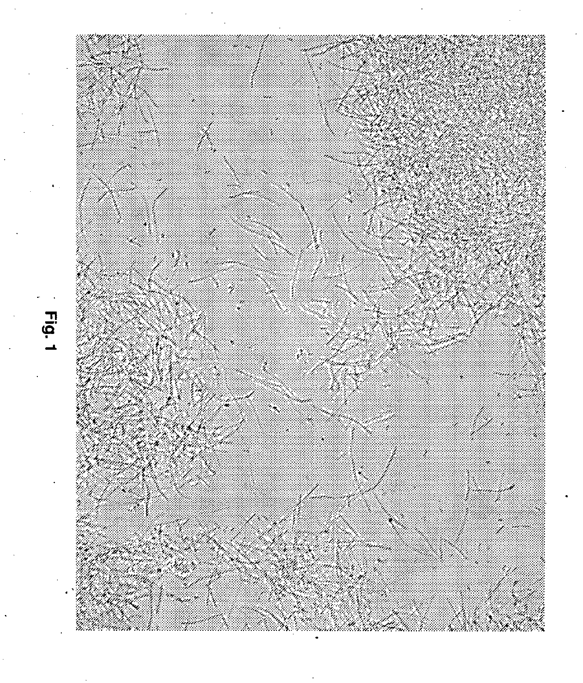

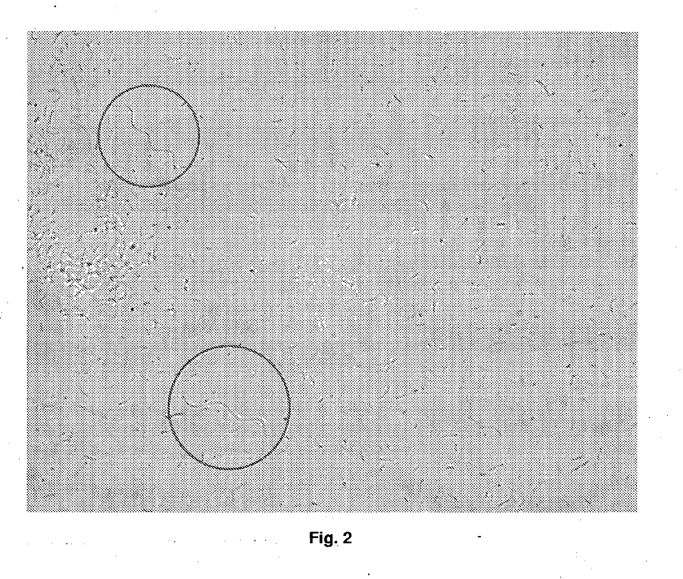

The drawing shows in the Fig. 1 photograph of culture medium 24 hours after inoculation of swab sample taken from the brewery equipment containing beside others bacteria of genus *Pectinatus,* and the Fig. 2 photograph of culture medium 24 hours after inoculation of the same swab sample from the brewery equipment.

### Examples of embodiment

A method for identification of bacteria of the genus *Pectinatus* in a sample of spoilt beer or in a sample taken from a brewery environment and/or from a brewery equipment according to the invention is based on usage of a culture medium which stimulates by its composition cultivation of anaerobic bacteria of genus *Pectinatus* and simultaneously inhibits cultivation of competing microorganisms. Upon simultaneous inoculation of bacteria of genus *Pectinatus* and lactic acid bacteria (which commonly happens in practice), then as early as after 24 hours it is possible to observe by means of common microscope the typical elongated cells of bacteria of genus *Pectinatus* with characteristic snake-like motion and to identify them safely according to these features. Lactic acid bacteria are at the same time by the composition of the used culture medium totally or at least partially inhibited, so that they do not obstruct in any way cultivation either identification of bacteria of genus *Pectinatus.* The used culture medium is pursuant to the need applicable as a sampling one, transport as well as a diagnostic one. Its composition is generally shown in the Table 1; at the same time its pH varies in the range from 5,5 to 5,9.

**Table 1.**

| | **Composition of culture medium used in a method according to the invention** | |
|---|---|---|
| *a* | Source of carbon, nitrogen, amino-acids and vitamins | MRS |
| *b* | Source of sulphur and biogenous metals | |
| *c* | Buffer component | |
| *d* | Component reducing the redox potential | |
| *e* | Iso-α-acids and/or their derivatives | |

The culture medium used in a method according to the invention may be easily and cheaply prepared by mixing the individual components *a* to *e* shown in the Table 1 at the required ratio. Nevertheless with respect to the fact, that the components *a*, *b* and *c* are in a suitable concentration contained already in the existing and commercially available non-selective culture medium of the MRS type, at least in some cases it is more advantageous if this non-selective culture medium is used as a base for culture medium used in the method according to the invention, to which the remaining components *d* and *e* are added. The non-selective culture medium of the MRS type besides the components *a*, *b* and *c* contains also other components, such as e.g. surface active substance - polyoxyethylene (20) sorbitan monooleate (commercially designated as Tween 80 or Polysorbate 80) in concentration of 1 g/l, and selective components simultaneously serving as a source of energy for some microorganisms - mixture of ammonium citrate in concentration of 2 g/l and CH₃COONa in concentration of 5 g/l, however their presence affects in no manner the desired effect of the culture medium used in the method according to the invention, either does not obstruct cultivation or identification of bacteria of genus *Pectinatus*. At other preparation procedures of the culture medium, these components need not to be used or substituted by other substances with the same or similar function.

The component *a* - a source of carbon, nitrogen, amino-acids and vitamins is either formed by one suitable substance, or more frequently by mixture of two or more various substances. If the non-selective culture medium of the MRS type is used as a base for culture medium, it is a mixture of peptone in concentration of 10 g/l, meat extract in concentration of 10 g/l, yeast extract in concentration of 5 g/l and glucose in concentration of 20 g/l. If, nevertheless the culture medium used in the method according to the invention is prepared by mixing the individual components, there may be used peptones, meat extract and yeast extract in any concentration in the range from 2 to 15 g/l, possibly other substances with the same or similar function, while e.g. peptone may be adequately replaced by tryptone, enzymatic casein hydrolysate, etc. Glucose may be used in any concentration from 1 to 25 g/l, possibly it may be replaced by fructose, lactic acid, etc.

The component *b* - a source of sulphur and biogenous metals, is formed either by one suitable substance or more frequently by a mixture of two, possibly even more various substances. In case of usage of the non-selective culture medium of the MRS type it is mixture of MgSO₄·7H₂O in concentration of 0,2 g/l and MnSO₄·4H₂O in concentration of 0,05 g/l. If the culture medium used in the method according to the invention is prepared by mixing the individual components, there may be used the same substances in concentration in the range from 0,025 to 0,25 g/l, possibly other substances with the same or similar function, e.g. FeSO₄.7H₂O and ZnSO₄.7H₂O in concentration in the range 0,005 - 0,02 g/l etc., or mixture of two or more suitable substances.

The component *c* - a buffer component, is usually formed by one substance. If the non-selective culture medium of the MRS type is used, then it is K₂HPO₄ in concentration of 2 g/l. If the culture medium used in the method according to the invention is prepared by mixing the individual components, there may be used K₂HPO₄ in concentration within the range from 1 to 3 g/l, possibly other substance with the same or similar function, e.g. Na₂HPO₄, etc., or mixture of two or more suitable substances.

The component *d* - a component reducing the redox potential, through its presence in the culture medium used in the method according to the invention reduces its redox potential, thus enabling cultivation of strictly anaerobic microorganisms, such as bacteria of genus *Pectinatus.* It is either one substance, mostly cysteine hydrochloride, ascorbic acid or sodium thioglycolate, whose concentration in the culture medium varies in the range from 0,25 to 1 g/l, or possibly mixtures of these substances, whose concentration in the culture medium varies from 0,25 g/l to 3 g/l, while concentration of any of the substances does not exceed the value of 1 g/l. In further variants may be for this purpose, besides the mentioned substances, also used other substances with the same or similar effects, possibly a mixture of such substances.

The component *e* - iso-α-acids and/or their derivatives, through its presence further stimulates cultivation of bacteria of genus *Pectinatus* and simultaneously totally or partially inhibits cultivation of the competing microorganisms. This group incorporates the iso-α-acids (mixture of trans- and cis- isomers) and their reduced hydrogenated derivatives, possibly their mixtures. As the most suitable seems to be usage of tetrahydroiso-α-acids, nevertheless besides them with the same results may be also used dihydroiso-α-acids or hexahydroiso-α-acids, mixtures of these reduced dehydrogenated derivatives of iso-α-acids, or any mixtures of iso-α-acids and their reduced hydrogenated derivatives. Concentration of this component in the culture medium used in the method according to the invention varies within the range from 10 to 80 mg/l, while preferably reaches 50 mg/l.

Another component of the culture medium , which is used only in some of its variants is agar. Its presence in the culture medium inhibits its oxidation during manipulation or transport and so it facilitates creating conditions suitable for existence of anaerobic microorganisms. The culture medium used in the method according to the invention, which is designated expressly for direct application in laboratory nevertheless do need not to contain agar at all.

To verify efficiency of culture medium used in the method according to the invention, a number of comparative experiments was performed, at which the non-selective culture medium of the MRS type and several variants of culture medium used in the method according to the invention created by combination of non-selective culture medium of the MRS type and components *d* a *e* in various concentrations was used. The particular composition of the culture medium tested is shown in the Table 2.

**Table 2.**

| **Component** | **Substance** | **Quantity of components of culture medium in 1000 ml of distilled water** | | | | | |
|---|---|---|---|---|---|---|---|
| | | **A** (MRS) | **B** | **C** | **D** | **E** | **F** |
| *a* | Peptone | 10,0 g | 10,0 g | 10,0 g | 10,0 g | 10,0 g | 10,0 g |
| | Meat extract | 10,0 g | 10,0 g | 10,0 g | 10,0 g | 10,0 g | 10,0 g |
| | Yeast extract | 5,0 g | 5,0 g | 5,0 g | 5,0 g | 5,0 g | 5,0 g |
| | Glucose | 20,0 g | 20,0 g | 20,0 g | 20,0 g | 20,0 g | 20,0 g |
| - | Tween 80 (Polyoxyethylene (20) sorbitane monooleate) | 1,0 g | 1,0 g | 1,0 g | 1,0 g | 1,0 g | 1,0 g |
| - | Ammonium citrate | 2,0 g | 2,0 g | 2,0 g | 2,0 g | 2,0 g | 2,0 g |
| | CH₃COONa | 5,0 g | 5,0 g | 5,0 g | 5,0 g | 5,0 g | 5,0 g |
| *b* | MgSO₄·7H₂O | 0,2 g | 0,2 g | 0,2 g | 0,2 g | 0,2 g | 0,2 g |
| | MnSO₄·4H₂O | 0,05 g | 0,05 g | 0,05 g | 0,05 g | 0,05 g | 0,05 g |
| *c* | K₂HPO₄ | 2,0 g | 2,0 g | 2,0 g | 2,0 g | 2,0 g | 2,0 g |
| *d* | Cysteine hydrochloride | - | 0,25 g | 0,25 g | 0,25 g | 0,25 g | 0,5 g |
| | Sodium thioglycolate | - | 0,25 g | 0,25 g | 0,25 g | 0,25 g | 0,5 g |
| *e* | Tetrahydroiso-α-acids | - | - | 10 mg | 35 mg | 50 mg | 50 mg |
| - | Agar | - | 0,1-0,2% | 0,1-0,2% | 0,1-0,2% | 0,1-0,2% | 0,1-0,2% |

First, effect of various concentrations of components *d* and e in culture medium used in the method according to the invention on cultivation of the most common bacteria of genus *Pectinatus* was monitored.

### Example1

The components of culture medium in variants A to F according to the Table 2 were stirred in 1000 ml of distilled water and the resultant solution was in portions of 10 ml poured into bacteriological test-tubes, which were subsequently sterilised in autoclave at the temperature of 121 °C for a period of 15 minutes. After cooling down, was into all variants of culture medium in bacteriological test-tubes inoculated 0,1 ml of dense suspension of the three collection strains of bacteria of genus *Pectinatus,* namely *Pectinatus frisingensis* DSM 20465, *Pectinatus frisingensis* CCM 6217 and *Pectinatus sp.* RIBM 2-86. After 24 hours of incubation at the temperature of 28 °C, creation of haze and sediment was evaluated and microscopy of each variant of culture medium A to F was performed.

No haze was observed in the variant A of the culture medium (MRS). During microscopy only randomly presence of very small number of bacteria of genus *Pectinatus* was observed.

A strong haze, which is an accompanying effect of presence of bacteria of genus *Pectinatus,* was observed in the variant B of the culture medium. Upon microscopy there was verified a good cultivation of these bacteria, which were without any further action easily and safely identifiable based on their typical snake-like shape and motion.

A strong haze was observed in the variants C to F of the culture medium. Microscopy confirmed very good cultivation of bacteria of genus *Pectinatus,* which were easily and safely identifiable based on their typical snake-like shape and motion.

Subsequently, effect of various concentrations of components *d* and *e* in culture medium to cultivation of the most common strains of lactic acid bacteria was observed.

### Example 2

Variants of culture medium A to F were prepared in the same method as in example 1. Into all variants of culture medium 0,1 ml of dense suspension of 25 collection strains of the most common lactic acid bacteria was inoculated. In the given case, the bacteria strains were the strains of genus *Lactobacillus* from the collection of VÚPS. After 24 hours of incubation at the temperature of 28 °C, creation of haze and sediment was evaluated and microscopy of each variant of culture medium A to F was performed.

A strong haze, which is an accompanying effect of presence of lactic acid bacteria, was observed in the variant A of the culture medium (MRS). During microscopy, a good cultivation of all strains of lactic acid bacteria was confirmed.

A strong haze was also observed in the variant B of the culture medium. Upon microscopy a good cultivation of all strains of lactic acid bacteria was confirmed.

A strong haze was observed in 19 test-tubes with the culture medium of variant C. After then, upon microscopy cultivation of 19 strains of lactic acid bacteria was confirmed. When compared with culture medium in variant A and B, by composition of culture medium 6 strains of lactic acid bacteria were inhibited.

A strong haze was observed in 10 test-tubes with the culture medium of variant D.. After then, upon microscopy cultivation of only 10 strains of lactic acid bacteria was verified. When compared with culture medium in variant A and B, by composition of culture medium 15 strains of lactic acid bacteria were inhibited.

No haze was observed in the variants E and F of the culture medium. Upon microscopy cultivation of none inoculated lactic acid bacteria strains was confirmed. Thus, all 25 lactic acid bacteria strains were inhibited by composition of the culture medium.

Next to this, there was monitored effect of various concentrations of components *d* and *e* in the culture medium to parallel cultivation of bacteria of genus *Pectinatus* and lactic acid bacteria, and to the identification possibilities of bacteria of genus *Pectinatus* upon microscopy.

### Example 3

Variants of culture medium A to F were prepared in the same method as in example 1. Into all variants of culture medium 0,1 ml of dense suspension of bacteria strain of genus *Pectinatus sp.* RIBM 2-86 according to the example 1, and simultaneously 0,1 ml of dense suspension of 25 collection strains of lactic acid bacteria mentioned in example 2 were inoculated. After 24 hours of incubation at the temperature of 28 °C creation of haze and sediment was evaluated and microscopy of each variant of culture medium A to F was performed.

A strong haze was observed in variant A of the culture medium (MRS). Upon microscopy a very good cultivation of all lactic acid bacteria strains was verified. The bacteria of genus *Pectinatus* were not observed at all.

A strong haze was observed in variant B of the culture medium. Upon microscopy a good cultivation of all lactic acid bacteria strains was verified, at the same time randomly also bacteria of genus *Pectinatus* were observed.

A strong haze was observed in variants C to F of the culture media. Upon microscopy were quite obviously observed typical cells with snake-like shape as well as motion, which are characteristic features of bacteria of genus *Pectinatus,* on basis of which these bacteria may be identified quite surely. At the culture medium of variant C and D simultaneously also cultivation of some lactic acid bacteria strains occurred, but their presence did not obstruct safe identification of bacteria of genus *Pectinatus.* At variants E and F the bacteria of genus *Pectinatus* totally dominated in the sample.

In further experiments only variants C to F of culture medium were used, while the possibility of their application for cultivation and identification of bacteria of genus *Pectinatus* was tested directly in a sample of the spoilt beer.

### Example 4

The culture medium in variants C to F was prepared and sterilised, in the same manner as in example 1. Due to the fact that cultivation and subsequent identification of bacteria of genus *Pectinatus* was performed in laboratory, the culture medium did not contain agar in any of the variants.

From the bottom of the vessel with beer infected by bacteria of genus *Pectinatus* and other microorganisms 0,1 ml of beer was taken and pipetted into each of test-tubes with 10 ml of respective variant of culture medium. After 24 hours cultivation at the temperature of 28 to 30 °C microscopy was performed, at which bacteria of genus *Pectinatus* were safely identified on basis of the presence of cells with typical snake-like shape and motion in all variants of the culture medium. Through this, the possibility of application of the culture medium for cultivation and subsequent identification of bacteria of genus *Pectinatus* directly in the contaminated beer was verified.

At variants C to F of culture medium and at the variant B of the culture medium was further tested a possibility of cultivation and subsequent identification of bacteria of genus *Pectinatus* in swab samples taken directly from the brewery equipment, in which the existence of bacteria of genus *Pectinatus* was in advance verified through methods known from the prior art.

### Example 5

The culture medium in variants C to F and culture medium in variant B were prepared and sterilised in the same manner as in example 1. The swab samples taken in a brewery environment once being taken were inoculated into all variants of the culture medium and transported into laboratory. Microscopy was performed after 24 hours of cultivation at the temperature of 28 to 30 °C.

In the culture medium of variant B various microorganisms typical for brewery environment, especially yeast and further bacteria of round or oblong shape were present. Some of the moving bacteria of oblong shape could at the same time be considered to be the bacteria of genus *Pectinatus,* but their identification was not reliable enough, as these bacteria did not show a typical snake-like motion.

For illustration, the Fig. 1 represents a photo from microscope with 630 times magnification.

In the culture medium in variants C to F nevertheless obviously dominated the typical bacteria with snake-like shape and motion, which could quite surely be identified as bacteria of genus *Pectinatus.* At the same time most of other microorganisms was inhibited, or their presence did not obstruct identification.

For illustration, the Fig. 2 represents a photo from microscope with 630 times magnification obtained at microscopy of culture medium in variant F.

From the experiments performed results, that the culture medium which comprises basic components such as a source of carbon, a source of nitrogen, a source of amino-acids, a source of vitamins, a source of sulphur, a source of biogenous metals and a buffer component with addition of small quantity of specific components, such as substances reducing redox potential (in the given case mixture of 0,25 g of cysteine hydrochloride and 0,25 g of sodium thioglycolate) and reduced dehydrogenated derivative of iso-α-acids (in the given case 10 mg of tetrahydroiso-α-acids), is applicable for identification of bacteria of genus *Pectinatus* both at direct inoculation of the pure culture of these bacteria, and at inoculation of culture comprising a mixture of various species of bacteria obtained from a spoilt beer or from swab samples taken in a brewery environment and/or from the brewery equipment. Identification of bacteria of genus *Pectinatus* by usage of culture medium described above at the same time may be done after only 24 hours from inoculation, so that this is many times quicker than to date used techniques. Moreover, this method does not require acquisition of special laboratory equipment, as bacteria of genus *Pectinatus* may be reliably identified upon microscopy of the culture medium using a common optical microscope.

In further variants the culture medium used in the method according to the invention may comprise other auxiliary components, which nevertheless are not substantial for its function. These are for example sources of salts such as e.g. NaCl, KCl, etc. in concentration of 1 to 3 g/l, acidobasic indicators, like e.g. chlorophenol red in concentration of 0,05 - 0,1 g/l, oxidation-reducing indicators, like e.g. methylene blue, resazurine etc. in concentration of 0,001 - 0,003 g/l, etc.

To achieve the best results in identification of bacteria of genus *Pectinatus* it is further advantageous if the swab samples from the brewery plant and/or from equipment, possibly from other places, are taken using the swabs, whose sampling head is before taking the sample dipped into the sterile destilled water with addition of a substance reducing the redox potential in concentration within the range from 0,25 to 1 g/l, possibly a mixture of several such substances in concentration 0,25 to 3 g/l, while concentration of none of these substances does exceed the value of 1 g/l. Preferably the substance reducing redox potential is cysteine hydrochloride, though usable are also other substances with the same effect, for example sodium thioglycolate, ascorbic acid, etc. possibly a mixture of such substances. The swab sample taken is immediately positioned into the culture medium of described composition.

## Claims

1. Use of a culture medium for identification of bacteria of the genus *Pectinatus* in a sample of spoilt beer or in a sample taken from a brewery environment and/or from a brewery equipment, wherein said culture medium comprises at least one source of carbon, at least one source of nitrogen, at least one source of amino-acids, at least one source of vitamins, at least one source of sulphur, at least one source of biogenous metals, at least one substance reducing redox potential, a buffer component, **characterized in that** said culture medium further comprises iso-α-acids and/or their reduced hydrogenated derivatives in a concentration of 10 to 80 mg/l.

2. Use according to the claim 1, **characterised in that**, the culture medium comprises iso-α-acids and/or their reduced hydrogenated derivatives in concentration of 50 mg/l.

3. Use according to the claim 1 or 2, **characterised in that**, the culture medium further comprises 0,05 to 0,3 % by weight of agar.

4. Use according to any of the claims 1 to 3, **characterised in that**, the culture medium comprises as reduced hydrogenated derivatives of iso-α-acids tetrahydroiso-α-acids.

5. Use according to any of the claims 1 to 4, **characterised in that**, the culture medium comprises as reduced hydrogenated derivatives of iso-α-acids dihydroiso-α-acids.

6. Use according to any of the claims 1 to 5, **characterised in that**, the culture medium comprises as reduced hydrogenated derivatives of iso-α-acids hexahydroiso-α-acids.

7. Method for identification of bacteria of the genus *Pectinatus* in a sample of spoilt beer, said method comprising innoculaton of the sample of spoilt beer into a culture medium which comprises at least one source of carbon, at least one source of nitrogen, at least one source of amino-acids, at least one source of vitamins, at least one source of sulphur, at least one source of biogenous metals, at least one substance reducing redox potential, a buffer component **characterized in that** said culture medium further comprises iso-α-acids and/or their reduced hydrogenated derivatives in concentration of 10 to 80 mg/l.

8. Method for identification of bacteria of the genus *Pectinatus* in a swab sample taken from a brewery environment and/or from a brewery equipment said method comprising:
- dipping the head of a swabinto the solution of sterilised destilled water with addition of a substance reducing the redox potential in a concentration of 0,25 to 3 g/l, and then taking the swab sample
- positioning the swab sample into a culture medium which comprises at least one source of carbon, at least one source of nitrogen, at least one source of amino-acids, at least one source of vitamins, at least one source of sulphur, at least one source of biogenous metals, at least one substance reducing redox potential, a buffer component **characterized in that** said culture medium further comprises iso-α-acids and/or their reduced hydrogenated derivatives in concentration of 10 to 80 mg/l.

9. The method according to the claim 8, **characterised in that**, the component reducing the redox potential is a substance from the group of cysteine hydrochloride, sodium thioglycolate, ascorbic acid in concentration of 0,25 to 1 g/l, or a mixture of at least two of these substances, while at the same time concentration of none of them exceeds value of 1 g/l.

## Patentansprüche

1. Verwendung des Kulturbodens zur Identifizierung der Bakterien der Gattung *Pectinatus* in der Probe des verdorbenen Biers oder in der Probe, die dem Brauereiumfeld und/oder der Brauereieinrichtung entnommen wurde, wobei der genannte Kulturboden mindestens eine Quelle des Kohlenstoffs, mindestens eine Quelle des Stickstoffs, mindestens eine Quelle der Aminosäuren, mindestens eine Quelle der Vitamine, mindestens eine Quelle des Schwefels, mindestens eine Quelle der biogenen Metalle, mindestens einen das jeweilige Redoxpotential herabsetzenden Stoff und eine puffernde Komponente enthält, **dadurch gekennzeichnet, dass** der genannte Kulturboden ferner Iso-α-Säuren und/oder ihre reduzierten hydrierten Derivate in der Konzentration von 10 bis 80 mg/l enthält.

2. Verwendung nach dem Anspruch 1, **dadurch gekennzeichnet, dass** der Kulturboden Iso-α-Säuren und/oder ihre reduzierten hydrierten Derivate in der Konzentration von 50 mg/l enthält.

3. Verwendung nach dem Anspruch 1 oder 2, **dadurch gekennzeichnet, dass** der Kulturboden ferner 0,05 bis 0,3 Gewichtsprozent von Agar enthält.

4. Verwendung nach einem der beliebigen Ansprüche 1 bis 3, **dadurch gekennzeichnet, dass** der Kulturboden als reduzierte hydrierte Derivate der Iso-α-Säuren die Tetrahydro-Iso-α-Säuren enthält.

5. Verwendung nach einem der beliebigen Ansprüche 1 bis 4, **dadurch gekennzeichnet, dass** der Kulturboden als reduzierte hydrierte Derivate der Iso-α-Säuren die Dihydro-Iso-α-Säuren enthält.

6. Verwendung nach einem der beliebigen Ansprüche 1 bis 5, **dadurch gekennzeichnet, dass** der Kulturboden als reduzierte hydrierte Derivate der Iso-α-Säuren die Hexahydro-Iso-α-Säuren enthält.

7. Verfahren zur Identifizierung der Bakterien der Gattung *Pectinatus* in der Probe des verdorbenen Biers, die die Inokulation der Probe des verdorbenen Biers in den Kulturboden einschließt, der mindestens eine Quelle des Kohlenstoffs, mindestens eine Quelle des Stickstoffs, mindestens eine Quelle der Aminosäuren, mindestens eine Quelle der Vitamine, mindestens eine Quelle des Schwefels, mindestens eine Quelle der biogenen Metalle, mindestens einen das jeweilige Redoxpotential herabsetzenden Stoff und eine puffernde Komponente enthält, **dadurch gekennzeichnet, dass** der genannte Kulturboden ferner Iso-α-Säuren und/oder ihre reduzierten hydrierten Derivate in der Konzentration von 10 bis 80 mg/l enthält.

8. Verfahren zur Identifizierung der Bakterien der Gattung *Pectinatus* im Abstrich, der dem Brauereiumfeld und/oder der Brauereieinrichtung entnommen wurde, der einschließt:
- Eintauchen des Entnahmeteils des Entnahmestabes in die Lösung des sterilen destillierten Wassers mit Zusatz des das Redoxpotential herabsetzenden Stoffes in der Konzentration von 0,25 bis 3 g/l, und Entnahme des Abstrichs
- Platzierung des Abstrichs in den Kulturboden, der mindestens eine Quelle des Kohlenstoffs, mindestens eine Quelle des Stickstoffs, mindestens eine Quelle der Aminosäuren, mindestens eine Quelle der Vitamine, mindestens eine Quelle des Schwefels, mindestens eine Quelle der biogenen Metalle, mindestens einen das jeweilige Redoxpotential herabsetzenden Stoff und eine puffernde Komponente enthält, **dadurch gekennzeichnet, dass** der genannte Kulturboden ferner Iso-α-Säuren und/oder ihre reduzierten hydrierten Derivate in der Konzentration von 10 bis 80 mg/l enthält.

9. Verfahren nach dem Anspruch 8, **dadurch gekennzeichnet, dass** die das Redoxpotential herabsetzende Komponente ein Stoff der Gruppe Zystein Hydrochlorid, Natriumtioglykolat, Ascorbinsäure in der Konzentration von 0,25 bis 1 g/l, oder die Mischung von mindestens zwei von diesen Stoffen ist, wobei die Konzentration keines von ihnen den Wert von 1 g/l übersteigt.

## Revendications

1. L'utilisation de la terre de culture pour identifier la bactérie du genre *Pectinatus* dans l'échantillon de la bière avariée ou dans l'échantillon retiré du milieu de la brasserie et/ou du dispositif de la brasserie, en temps que la terre de culture mentionnée comprend au moins une source de carbone, au moins une source d'azote, au moins une source d'acides aminés, au moins une source de vitamines, au moins une source du soufre, au moins une source de métaux biogènes, au moins une substance abaissant le rédoxpotenciel et le composant de la solution-tampon, **caractérisé en ce que** la terre de culture mentionnée comprend encore les iso-α-acides et/ou leurs produits dérivés réduits hydrogènes dans la concentration de 10 à 80 mg/l.

2. L'utilisation suivant la revendication 1, **caractérisé en ce que** la terre de culture comprend les iso-α-acides et/ou leurs produits dérivés réduits hydrogènes dans la concentration de 50 mg/l.

3. L'utilisation suivant la revendication 1 ou 2, **caractérisé en ce que** la terre de culture comprend encore de 0,05 à 0,3% de masse des agars.

4. L'utilisation suivant quelconque des revendication de 1 à 3, **caractérisé en ce que** la terre de culture comprend comme produits dérivés réduits hydrogènes des iso-α-acides de l'acide tetrahydroiso-α.

5. L'utilisation suivant quelconque des revendication de 1 à 4, **caractérisé en ce que** la terre de culture comprend comme produits dérivés réduits hydrogènes des iso-α-acides de l'acide dihydroiso-α.

6. L'utilisation suivant quelconque des revendication de 1 à 5, **caractérisé en ce que** la terre de culture comprend comme produits dérivés réduits hydrogènes des iso-α-acides de l'acide hexahydroiso-α.

7. Le mode de l'identification des bactéries du genre *Pectinatus* dans l'échantillon de la bière avariée qui comprend l'inoculation de l'échantillon de la bière avariée dans la terre de culture, qui comprend au moins une source de carbone, au moins une source d'azote, au moins une source d'acides aminés, au moins une source de vitamines, au moins une source du soufre, au moins une source de métaux biogènes, au moins une substance abaissant le rédoxpotenciel et le composant de la solution-tampon, **caractérisé en ce que** la terre de culture mentionnée comprend encore les iso-α-acides et/ou leurs produits dérivés réduits hydrogènes dans la concentration de 10 à 80 mg/l.

8. Le mode de l'identification des bactéries du genre *Pectinatus* dans la crasse prélevée dans le milieu de la brasserie et/ou du dispositif de la brasserie, qui comprend:
- l'enfoncement de la partie de prélèvement du baton de prélèvement dans la solution de l'eau stérile distilée avec l'addition de la substance diminuante le rédoxpotenciel dans la concentration de 0,25 à 3 g/l, et le prélèvement de la crasse;
- le placement de la crasse dans la terre de culture, qui comprend au moins une source de carbone, au moins une source d'azote, au moins une source d'acides aminés, au moins une source de vitamines, au moins une source du soufre, au moins une source de métaux biogènes, au moins une substance abaissant le rédoxpotenciel et le composant de la solution-tampon, **caractérisé en ce que** la terre de culture mentionnée comprend encore les iso-α-acides et/ou leurs produits dérivés réduits hydrogènes dans la concentration de 10 à 80 mg/l.

9. Le mode suivant la revendication 8, **caractérisé en ce que** le composant abaissant le rédoxpotenciel est la substance du groupe de la cystéine chlorhydrate, thioglycolate sodique, l'acide ascorbique dans la concentration de 0,25 à 1 g/l, ou le mélange au moins de ces deux substances, en temps que la concentration de chacune d'elles ne dépasse pas la valeur de 1 g/l.
